# EUROPEAN PATENT APPLICATION

(11) **EP 4 763 155 A1**
(43) Date of publication of application: **24.06.2026**
(21) Application number: 25226057.5
(22) Date of filing: 19.12.2025
(51) Int. Cl.: A61F 5/453, A61F 5/455

(54) **URINE COLLECTION DEVICE**

(30) Priority: 20.12.2024 US 202463737053 P
(71) Applicant: Wellspect AB, 431 21 Mölndal (SE)
(72) Inventor: LOVMAR, Martin, 431 21 Mölndal (SE); JOHANSSON, Mathilda, 431 21 Mölndal (SE); BENNERSTEN, Viktor, 431 21 Mölndal (SE); CARTER, Mary, Arlington, 02474 (US); LIEBLER, Sara, Watertown, 02472 (US)
(74) Representative: Taor, Simon Edward William

(57) **Abstract**

The present disclosure relates to a urine collection device. The urine collection device comprises a liquid impermeable layer at least partially defining an interior volume, wherein the liquid impermeable layer comprises a urine inlet opening and a collection region. The urine collection device further comprises a conduit comprising a first opening being in communication with the urine collection region, and an absorbent material arranged inside the interior volume and extending from the urine inlet opening towards the urine collection region to define a low flow urine transportation path. The urine collection device further comprises a high flow urine transportation path extending from the urine inlet opening to the urine collection region, wherein a urine flow rate per unit area is higher for the high flow urine transportation path compared to the low flow urine transportation path.

## Description

### TECHNICAL FIELD OF THE INVENTION

The present invention relates to a urine collection device for external urine collection. The general principles proposed may be used for any type of external urine collection device. Some embodiments of the present invention relate specifically to a urine collection device suitable for male users and some embodiments relate specifically to a urine collection device suitable for female users.

### BACKGROUND

Traditional urinary catheters for emptying a user's bladder are realized using a hollow catheter shaft provided with one or more drainage openings in one end and a holder part with an outlet in the other end. The urinary catheter is inserted into the bladder of the user (typically via the urethra) whereby urine can be voided from the bladder via the urinary catheter. A urinary catheter may be an intermittent catheter configured for intermittent use during a short period of time and is typically configured to be used only once, to empty the bladder and subsequently discarded. A urinary catheter may also be an indwelling catheter (Foley catheter) which is adapted for long time use wherein the indwelling catheter is configured to remain in the bladder for continuous drainage over a long period of time, such as several hours, most of a day or even longer.

While urinary catheters have been continuously developed over the last decades to make them more effective, more convenient to use, easier to store and more sustainable, the fact that a catheter shaft must be inserted into the user makes urinary catheters inappropriate to use in some scenarios. Additionally, a major problem with indwelling urinary catheters is that these may cause urinary tract infections. Furthermore, some indwelling urinary catheters may be uncomfortable to use for long periods of time, making them ill-suited for e.g. long term treatment of urinary incontinence. The fact that many types of indwelling catheters require the assistance of a medical professional for insertion and operation also makes them inconvenient to use in some cases.

To this end, various external devices for urine collection have been proposed, including an impermeable housing, configured for external placement against an area surrounding the urethra of a user. A conduit supplying suction is arranged to communicate with the inside of the impermeable housing so as to withdraw any urine expelled from the user. In some implementations, an absorbent material is further used to fill the impermeable housing to e.g. improve urine absorption and make the device more comfortable to use. However, a drawback with existing urine collection devices is that they are not able to handle large variations in urine flow rate. Typically, urine collection devices are designed to transport a constant and slow flow of urine, and while this works well for some users, the urine flow rate for some users fluctuates whereby the urine collection device at times becomes oversaturated and loses its urine collection capability, leading to urine leakage. To this end, there is a need for a new and improved urine collection device for urine collection, which overcomes at least some of the drawbacks mentioned above.

### SUMMARY

It is the purpose of the present disclosure to present an improved urine collection device which is capable of handling large variations in urine flow while still maintaining its urine collection capability.

According to an aspect of the present invention, there is provided a urine collection device comprising a liquid impermeable layer at least partially defining an interior volume. The liquid impermeable layer comprises a urine inlet opening and a urine collection region. The urine collection device further comprises a conduit having a first open end, wherein the first open end of the conduit is in communication with the collection region of the interior volume. The urine collection device further comprises an absorbent material arranged inside the interior volume and extending from the urine inlet opening towards the urine collection region so as to define a low flow urine transportation path. The urine collection device further comprises a high flow urine transportation path extending from the urine inlet opening to the urine collection region, wherein the high flow urine transportation path is configured to allow a higher urine flow rate per unit area compared to the low flow urine transportation path.

The urine collection device according to the aspect of the present invention is adapted to be held against the user such that the urine inlet opening faces the urethral opening of the user. As will be described below, the urine collection device may also be used by users with a penis, in which case the penis may be inserted through the urine inlet opening and into the high flow urine transportation path.

Urine which is deliberately or not deliberately voided by the user, or urine which leaks from a user suffering from urinary incontinence, enters into the interior volume defined by the liquid impermeable layer and may then be collected in the urine collection region thereof. The urine can then be removed via the conduit by providing suction through the conduit. In this way, urine can effectively be collected from the user without the need for e.g. catheterization or repeatedly changing diapers.

Additionally, since two distinct urine transportation paths, one high flow transportation path and one low flow transportation path, are present at the interface defined by the urine inlet opening, the urine collection device's ability to handle different types of urine flow without leakage is greatly enhanced. The low flow urine transportation path excels in handling slow flows of urine, e.g. a continuous low flow or trickling/dripping flow. Such slow flows are typically not ejected from the urethra with high pressure but rather flow out of the urethra and along the skin the of the user. With the low flow urine transportation path, urine flowing along the skin of the user may still be absorbed into the low flow urine transportation path, whereby the urine by means of gravity, means of wicking through the absorbent material and/or due to the suction pressure provided through the conduit, is transported to the urine collection region and/or drained into the high flow urine transportation path.

On the other hand, larger urine flows such as bursts, gushes, or direct voiding of urine by the user could, at least locally, saturate the absorbent material since large urine volumes cannot be rapidly transported via the absorbent material of the low flow urine transportation path. To this end, the high flow urine transportation path, which is free from highly absorbent material and which extends from the urine inlet opening to the urine collection region, is provided so as to effectively collect these types of larger urine flows. Since the high flow urine transportation path may be free from absorbent material, larger volumes of urine can be transported much more rapidly, whereby the risk of saturation of the absorbent material, and leakage, can be avoided even for large urine flows.

Hence, the urine collection device of the first aspect excels in handling different magnitudes of urine flow while preventing leakage.

The absorbent material may be a wicking material. A wicking material is capable of transporting a liquid by means of e.g. capillary forces which serve to transport a liquid through the material.

The impermeable layer may be a layer which is separate from the other parts of the urine collection device. For example, an impermeable layer is wrapped around the other parts of the urine collection device. The impermeable layer may be attached to the other parts e.g. by means of friction, using an adhesive and/or welding. It is also envisaged that the impermeable layer is coating which is sprayed and/or painted onto the other parts of the urine collection device, wherein the coating forms a fluid impermeable layer upon setting.

A urine collection device with a high-flow urine transportation path and a low flow urine transportation path may be beneficial for use by both males and females. Some urine collection devices are specifically designed for male users (or any user with a penis) and some urine collection devices are specifically designed for female users (or any user with a retracted or buried penis) as described below.

In some implementations, the high flow urine transportation path comprises at least one open (e.g. air-filled) channel extending from the urine inlet opening to the urine collection region. The open fluid channel may be defined by at least one of a urine guiding body, a fluid channel extending through a porous material, and a path provided through the absorbent material.

The high flow urine transportation path may be realized by leaving a path which is free from absorbent material, i.e. the path may be air-filled, leading from the urine collection region (from the first open end of the conduit) to the urine inlet opening. However, there are other ways in which the high flow urine transportation path may be formed.

For example, in some implementations, the high flow urine transportation path is at least partially filled with a porous material.

The porous material has a large number of pores forming channels through which the urine can flow rapidly. The porous material may have a high permeability and large pores while, at the same time, it does not absorb large amounts of urine. For example, the porous material may be a filter foam material. The porous material may be made of polymer material, such as a plastic material. The porous material preferably exhibits high permeability and porosity while preferably exhibiting low absorption so as to allow urine to pass through. The porous material may be a 3D mesh structure. The porous material may be rigid or semi-rigid. For example, the porous material is hydrophobic. Hydrophobic material properties in combination with large pores may allow large volumes of urine to be transported rapidly through the porous material.

In some implementations the porous material is non-absorbent.

By non-absorbent, it is meant that the porous material has a liquid absorption capacity which is less than that of the absorbent material. As the purpose of the porous material is to allow urine to pass through rapidly, it is beneficial if the porous material does not absorb too much urine. Accordingly, while the high flow urine transportation path may be filled with a material which exhibits some absorption, it is much less absorptive than the absorbent material of the low flow urine transportation path. As an example, the porous material has an absorption capacity of less than 80% or less than 50% of the absorption capacity of the absorbent material, when the absorption capacity is measured as a mass liquid per volume material (e.g. grams liquid per cubic decimeter material). For example, a material with an absorption capacity of 30 g per cubic decimeter material absorbs fluid weighing 30 g in each cubic decimeter of material. Since some absorbent materials shrink and/or expand when absorbing liquid the material volume may be established in the dry state, prior to liquid absorption.

In some implementations, the porous material comprises a plurality of loose bodies such as a plurality of beads made of e.g. a plastic material. If the loose bodies are arranged along a path so as to form a path, urine may effectively flow around the beads and rapidly reach the urine collection region.

In some implementations, the high flow urine transportation path is at least partially defined by an air-filled space.

For example, the absorbent material forming the low flow urine transportation path may be provided with a slit or groove which defines the high flow urine transportation path.

In some implementations, the urine collection region is at least partially filled with the porous material. As urine can flow rapidly through the porous material, the urine collection region may be at least partially (even fully) filled with porous material. The urine can still reach the first open end of the conduit rapidly. The high flow urine transportation path and urine collection region may overlap. The urine collection region, like the high flow urine transportation path, may additionally or alternatively be defined by an air-filled space or by a urine guiding body.

In some implementations, the impermeable layer comprises an impermeable film layer. For example, the impermeable film layer is a plastic film layer. A benefit with a film layer is that this allows the urine collection device to be flexible and deformable so as to better conform to the shape of the user's body.

The interior surface and/or exterior surface of the impermeable film layer may be laminated with a non-woven material. A non-woven lamination on the exterior surface makes the urine collection device soft and more comfortable to use. A non-woven lamination on the inside may increase friction and allow components housed therein to remain in place. The non-woven lamination on the interior surface may also contribute to urine wicking and form part of the low flow urine transportation path.

In some implementations, the urine collection device further comprises a urine guiding body arranged inside the interior volume, the urine guiding body forming a channel at least partially defining the high flow urine transportation path and/or urine collection region.

The urine guiding body may be flexible or rigid. For example, the urine guiding body is made of a polymer material such as plastic material. The urine guiding body may be made of a material impermeable to urine. The urine guiding body acts as scaffolding or "skeleton" which keeps the absorbent material and/or impermeable layer away from the high flow urine transportation path to allow urine to rapidly be transported through the high flow urine transportation path. For example, the urine guiding body is made from a mesh material.

In some implementations, a first portion of the conduit extends through the interior volume and wherein the urine guiding body is attached to the first portion of the conduit and extends at least partially along the first portion of the conduit.

In this way the urine collection device becomes easy to assemble since the urine guiding body is attached to the conduit directly. Furthermore, the urine guiding body may be configured together with the conduit to form the high flow urine transportation path.

In some implementations, the conduit and/or the urine guiding body are reusable. For instance, to reuse the urine guiding body, the urine guiding body may according to some implementations be removed from the conduit, cleaned or disinfected, and attached to a new or reused conduit. This reduces waste and makes the urine collection device more sustainable.

In some implementations, the urine guiding body comprises a receptacle arranged to face the urine inlet opening wherein the receptacle is configured to direct urine entering the receptacle to the channel.

By channeling or concentrating the urine with the receptacle, urine entering the urine inlet opening over a comparatively large area may be collected and concentrated to a comparatively small high flow urine transportation path. The receptacle will thus improve the chances of a high velocity and/or high volume urine flow entering directly into the high flow urine transportation path.

In some implementations, the absorbent material is a wicking material, configured to wick liquid, such as urine.

The absorbent material may preferably feature capillary motion properties or wicking properties (e.g. the absorbent material is a wicking material), which contributes to transporting urine away from the urine inlet opening and towards the urine collection region and/or to the high flow urine transportation path. The absorbent material may be based on cellulose and may e.g. be paper material similar in properties to a paper towel or napkin. The absorbent material may comprise cotton, hemp, or microfibers. The absorbent material may be a woven or non-woven material. Additionally or alternatively, the absorbent material may be a gauze, cloth or foam material. While most types of wicking materials will also feature some degree of absorption, the main purpose of the absorbent material is to transport the urine away from the urine collection opening, towards the urine collection region. Some absorption is of course desirable, to e.g. capture urine trickling along the skin of the user, but to enable long term use of the urine collection device without leakage or (local) saturation, a material with wicking properties is advantageous. For example, the absorbent material is a foam or non-woven material made from a polymer. In some implementations a porous material at least partially fills the high flow urine transportation path. In such implementations, the foam forming the absorbent material has a higher density than the porous material.

The absorbent material may comprise one or more synthetic polymers which have an absorbance depending on their hydrophilicity. Examples of hydrophilic polymers are polyacrylic acid (PAA), polyvinyl alcohol (PVA), polyethylene glycol (PEG), polyvinylpyrrolidone (PVP), polyacrylamide (PAAm), cellulose, polyurethane (e.g. with hydrophilic segments) and polyamide (nylon). These materials are hydrophilic and may be used to absorb liquid.

Examples of less hydrophilic, even hydrophobic, polymers are polyethylene terephthalate (PET), polystyrene (PS), polypropylene (PP), polyethylene (PE) and polytetrafluoroethylene (PTFE). The less hydrophilic polymers may be made absorbent using surface treatments or chemical modifications and may accordingly be used as the absorbent material after such treatment. The structure of the material may also affect the absorbent properties. For example, a non-woven made of PE or PP could be modified to form an absorbent material.

When the urine collection device is held against a user, the absorbent material may absorb urine even in situations where the urine trickles along the skin the of the user. By various effects (e.g. capillary forces, gravity and/or suction provided via the conduit) the urine will be pulled through the absorbent material so as to reach the urine collection region directly or drain into the high flow urine transportation path. As a consequence, the part of the absorbent material that is closest to the urine inlet opening may be continuously drained from urine to retain its absorption properties and avoid saturation for low to medium urine flow rates.

In some implementations, a permeable layer covers at least a portion of the urine inlet opening. The permeable layer may e.g. be realized with a net- or web-like structure. For example, the permeable layer is a web of polymer strands. The permeable layer may be made of a woven, non-woven or mesh material. The permeable layer may be made from an impermeable or permeable material provided with multiple openings for allowing urine to pass through. The main purpose of the permeable layer is to provide a screening effect that allows urine to pass through but prevents body parts of the user from passing through. For example, the permeable material has openings which have a largest dimension of 1 mm or less, 0.8 mm or less or 0.5 mm or less. By avoiding large openings, the permeable layer becomes more comfortable when held against the user's body.

In some implementations, the urine inlet opening is completely covered with a permeable layer.

That is, the permeable layer may cover the entire urine inlet opening whereby urine passes through the permeable material prior to reaching one of the two transportation paths. A benefit of covering the entire urine inlet opening with the same material is that the look and feel of the urine inlet opening may be homogenous across its surface, wherein the urine collection device may be more comfortable to use.

In some implementations, the interface between the low flow urine transportation path is covered with the permeable layer, wherein the permeable layer has an aperture and wherein the high flow urine transportation path communicates with the aperture of the permeable layer.

Hereby, the permeable layer may be used to retain the absorbent material whereby the high flow urine transportation path is free from the absorbent material. Using the permeable layer, it is easy to shape the absorbent material as desired, e.g. the absorbent material may be shaped to form a lip or rim surrounding the inlet to the high flow urine transportation path which facilitates formation of a tighter seal when the urine collection device is held against the user.

In some implementations, the permeable layer covers the interface between the high flow urine transportation path and the urine inlet opening and wherein a remaining area of the urine inlet opening is covered with the absorbent material.

Since the high flow urine transportation path may be at least partially air-filled (i.e. empty), defined by a urine guiding portion or by porous material, it could be beneficial to prevent the user, or a part of the user's body, from entering the high flow urine transportation path. For example, there could otherwise be a risk that a body part of the user gets stuck or pinched in the high flow urine transportation path or that the porous material is uncomfortable to touch.

In some implementations, the absorbent material comprises an absorbent layer at least partially surrounding the high urine flow transportation path and/or urine collection region.

Optionally, the absorption layer may completely circumferentially surround the high flow urine transportation path over at least a portion of the elongated extent of the urine collection device.

It is also envisaged that the absorbent material is realized using multiple layers, e.g. an outer absorbent layer covering and an intermediate absorbent layer or intermediate absorbent stuffing. Wherein the intermediate absorbent layer or intermediate absorbent stuffing is arranged between the outer absorbent layer and the high flow urine transportation path. Hereby, urine may be drained from the absorbent material into the high flow urine transportation path to enable efficient urine collection.

In some implementations, the first opening of the conduit communicates directly with the high flow urine transportation path and/or urine collection region.

The urine collection region may transition into the high flow urine transportation path. That is, the urine collection region may alternatively be free from absorptive material and/or may be at least partially filled with the porous material and/or air-filled. Irrespective of the urine collection region being filled, partially filled or free from porous material, the urine collection region is in close proximity to the opening of the conduit whereby urine is rapidly removed from the urine collection region using a suction pressure in the conduit.

In some implementations, the urine collection region is defined as a space in a bottom portion of the urine collection device in the vicinity of the first opening of the conduit and at least partially enclosed by the impermeable layer. The urine collection region may be enclosed by the impermeable layer in all but one direction allowing the high flow urine transportation path (and optionally low flow urine transportation path) to communicate with the urine collection region. The urine collection region may be at the opposite end of the urine collection device from the urine inlet opening, or at least in the opposite half portion the urine collection device from the urine inlet opening. In some implementations, the absorbent material does not communicate with the urine collection region but ends upstream of the urine collection region and drains into the high flow urine transportation path upstream of the urine collection region.

The urine collection region may also be referred to as a sump, i.e. a space in which urine is collected e.g. by the force of gravity, when the urine collection device is in use. While the urine collection device is capable of efficiently collecting urine in a variety of orientations, the urine collection region will be located in the bottom portion of the device when in use. The first opening of the conduit communicates with the urine collection region and to accomplish this, the conduit may enter the urine impermeable layer in the vicinity of the urine collection region or enter through an opposite (top) portion of the urine collection device and extend through the interior volume to reach the urine collection region.

In some implementations, the urine collection region and/or high flow urine transportation path is elongated and the absorbent material extends alongside the urine collection region and/or high flow urine transportation path a distance of at least 2 cm, at least 3 cm or at least 4 cm to facilitate drainage of the absorbent material into the urine collection region and/or high flow urine transportation path.

Accordingly, in some implementations, the urine collection device is elongated and extends along an elongation axis, and wherein a length of the high flow urine transportation and/or urine collection region along the elongation axis is at least 2 cm, at least 3 cm or at least 4 cm.

In some implementations, the high flow urine transportation path and/or urine collection region is at least partially surrounded by the low flow urine transportation path at the urine inlet opening.

There are many envisaged layouts of the absorbent, low flow urine transportation path and high flow urine transportation path at the urine inlet opening. However, by arranging the absorbent urine transportation path so as to at least partially, or fully, surround the inlet to the high flow urine transportation path, the risk for leakages is reduced since urine which does not enter directly into the high flow urine transportation path is absorbed by the absorbent material surrounding the entry to the high flow urine transportation path. In some implementations, the absorbent material interfacing with the urine inlet opening partially surrounds the inlet of the high flow urine transportation path and is generally U or V shaped. The region below and to the left and right side (when the urine collection device is arranged upright, with the urine collection region at the bottom) of the inlet to the high flow urine transportation path may be most important to cover to prevent leakages.

In some implementations, the high flow urine transportation path communicates with at least 20%, at least 30% or at least 40 % of an opening area of the urine inlet opening. Additionally or alternatively, the low flow urine transportation path communicates with at least 20%, at least 30% or at least 40 % of an opening area of the urine inlet opening.

The high flow urine transportation path is capable of rapidly transporting urine to the urine collection region, but it is not necessary for the inlet of this urine transportation path to occupy a large portion of the urine inlet opening area. For example, the inlet of the high flow urine transportation path occupies less than 50%, less than 40% or less than 30% of the urine inlet opening. For example, the inlet of the high flow urine transportation path occupies between 10% and 40% of the urine inlet opening area or between 10% and 30% of the urine inlet opening area.

In some implementations, the high flow urine transportation is surrounded by the absorbent material at the urine inlet opening. This may improve leakage prevention and make the urine collection device more comfortable to use.

In some implementations, the urine inlet opening is surrounded by an attachment element comprising an adhesive configured for attaching the urine collection device to a user. This allows the urine collection device to be attached to the user so as to be retained in a desirable position, while also further reducing the risk for urine leakage.

In some implementations, the high flow urine transportation path comprises an air-filled upstream portion and a downstream portion connecting to the urine collection region, wherein the air-filled upstream portion is configured to receive the shaft of a penis and wherein the downstream portion is at least one of air-filled, at least partially filled with a porous material, and comprises a urine guiding body.

This arrangement is especially suited for male users, or any user with a penis, since the penis may be inserted in the upstream air-filled part of the urine collection device wherein the downstream high flow urine transportation path is still capable of allow large urine flows to pass to the urine collection region. In some cases, at least the upstream portion of the high flow urine transportation path is surrounded with the absorbent material defining the low flow urine transportation path.

### BRIEF DESCRIPTION OF THE DRAWINGS

The present invention will be described in more detail with reference to the appended drawings, showing currently preferred embodiments of the invention.
Figures 1a-f show various side views and cross-sectional views of urine collection devices according to some implementations.
Figure 2 illustrates schematically how urine entering the interior volume of a urine collection device is transported to the urine collection region and subsequently drawn into the conduit, according to some implementations.
Figures 3a-e show various cross-sectional views of envisaged urine collection devices having two urine transportation paths, a high flow path and an absorption path.
Figures 4a-b show cross-sectional views of urine collection devices comprising a porous material at least partially defining the high flow urine transportation path.
Figures 5a-d show cross-sectional views of urine collection devices comprising a permeable layer at least partially covering the urine inlet opening.
Figures 6a-b show cross-sectional views of a urine collection device with a urine guiding body, according to some implementations.
Figure 7 shows a cross-sectional view of a urine collection device with an absorbent material formed as a sheet wrapped around a porous material at least partially defining the high flow urine transportation path, according to some implementations.
Figures 8a-f show various side views and cross-sectional views of a urine guiding body according to some implementations.
Figures 9a-d show a side view of a urine collection device where progressively more components are added to highlight how the urine collection device can be constructed according to some implementations.
Figures 10a-b illustrate schematically different layouts of the urine inlet opening with respect to how the absorbent material and inlet to the high flow urine transportation channel are arranged.
Figures 11a-d show cross-sectional views of envisaged urine collection devices, highlighting how urine is transported to the urine collection region.
Figures 12a-c show cross-sectional views of urine collection devices configured for a male user, or in general any user with a penis.
Figure 13 is a front view of a urine collection device configured for male user, or in general any user with a penis.

### DETAILED DESCRIPTION OF CURRENTLY PREFERRED EMBODIMENTS

In the following detailed description, preferred embodiments of the invention will be described. However, it is to be understood that features of the different embodiments are exchangeable between the embodiments and may be combined in different ways, unless anything else is specifically indicated. It may also be noted, for the sake of clarity, that the dimensions of certain components illustrated in the drawings may differ from the corresponding dimensions in real-life implementations of the invention.

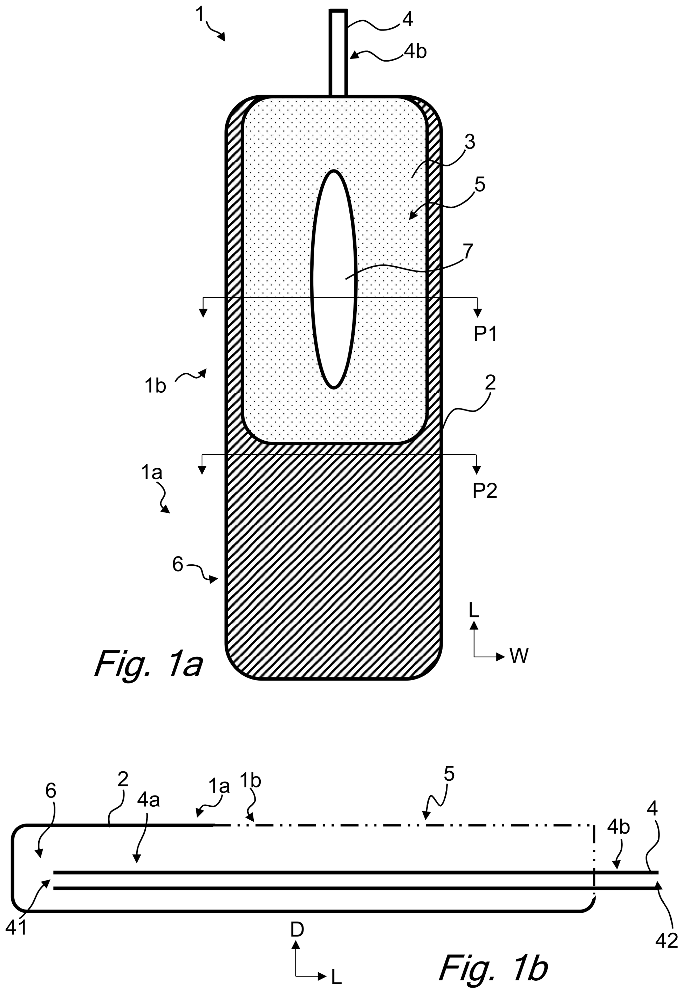

Fig. 1a shows a urine collection device 1 for collecting urine. The urine collection device 1 comprises a liquid impermeable layer 2 at least partially defining an interior volume. The liquid impermeable layer 2 is made of a material impermeable to urine so as to prevent urine from passing through the liquid impermeable layer 2. The liquid impermeable layer 2 may e.g. be made of a polymer material such as a plastic material. As one example, the liquid impermeable layer 2 is a plastic film. In some implementations, the impermeable layer is a film laminated on at least one of its interior surfaces (facing the interior volume) and exterior surfaces (facing the opposite direction) with a non-woven material. Notably, in embodiments where the exterior surface is laminated with a non-woven material, the exterior of the urine collection device will be softer and more comfortable to touch, which makes the urine collection device more comfortable to use. A non-woven lamination on the interior surface may contribute to formation of the low flow urine transportation path and/or contribute to increasing surface friction allowing other components (such as the absorbent material 3, any porous material or urine guiding body, and the conduit) to stay in their intended place. It also envisaged that the impermeable layer 2 is a coating painted and/or sprayed on the other parts of the urine collection device 1, wherein the coating becomes fluid impermeable upon setting or drying.

The liquid impermeable layer 2 may also be rigid or semi rigid. For instance, the liquid impermeable layer 2 is made from a thick plastic material, a rubber material or a silicone material.

The liquid impermeable layer 2 is shaped and/or folded so as to define an interior volume and a urine inlet opening 5, the urine inlet opening 5 being located in a top portion 1b of the urine collection device 1. This allows urine to enter the interior volume from the outside of the liquid impermeable layer 2, via the urine inlet opening 5. The liquid impermeable layer 2 further defines a urine collection region 6 in which urine having entered through the urine inlet opening 5 may be collected to be efficiently transported away via the conduit 4.

The exemplary urine collection device 1 shown in fig. 1a is elongated along an elongation axis parallel to the length axis L. The urine collection device 1 further has a width along the width axis W perpendicular to the length axis L. A benefit of the urine collection device 1 being elongated is that it may more easily and comfortably fit between the thighs or labia of a female user, or thighs of a user with a retracted or buried penis.

To form the urine collection region 6, the liquid impermeable layer 2 has been folded or shaped to form a cup shape or sump at a bottom portion 1a of the urine collection device 1. In fig. 1a the urine collection device 1 is depicted standing upright, but the urine collection device 1 is in general intended to be used in a variety of positions. For example the urine collection device 1 may be used laying down almost fully horizontally. The urine collection device 1 may be used effectively in a large number of orientations but preferably the urine collection region 6 and/or bottom portion 1a is always at the lowest part to collect urine for removal via the conduit 4. By placing the urine inlet opening 5 against the urethral opening of a user, any urine expelled by the user, or leaking from the user, will enter the interior volume via the urine inlet opening 5. Urine entering through the urine inlet opening may then travel to the urine collection region 6, which will generally be located in the bottom portion 1a of the urine collection device 1 which is arranged lowest during use, to allow gravity to assist in transporting urine to the urine collection region 6.

As will be described below, the urine collection device 1 has multiple transportation paths for transporting the urine from the urine inlet opening 5 towards the urine collection region 6.

With further reference to fig. 1b showing schematically a cross-section in a length-depth plane of the urine collection device 1 it is seen that the urine collection device 1 further comprises a conduit 4 extending into the interior volume defined by the liquid impermeable layer 2. The conduit 4 is a hollow element, e.g. a tube or similar, extending from a first opening 41 to a second opening 42. A first portion 4a of the conduit 4 comprising the first opening 41 may be arranged within the interior volume whereby a second portion 4b of conduit comprising the second opening 42 is arranged externally of the interior volume. The first opening 41 of the conduit 4 is arranged in communication with the urine collection region 6.

The second opening 42 of the conduit 4 may be configured to be connected to a vacuum source (e.g. directly or via an extension conduit) for providing a suction pressure that withdraws urine from the urine collection region 6. The urine withdrawn via the conduit 4 may be stored in a storage container and/or discarded.

To allow the conduit 4 to pass into the interior volume, the conduit 4 may extend through the urine inlet opening 5 as is the case in the embodiment of fig. 1b. To make the urine collection device 1 convenient to use, it may be beneficial for the conduit 4 to exit the interior volume in the top portion 1b in a direction perpendicular to the depth and width axes D, W and parallel to the length axis L. To this end, the urine inlet opening 5 may extend over a top end of the urine collection device 1.

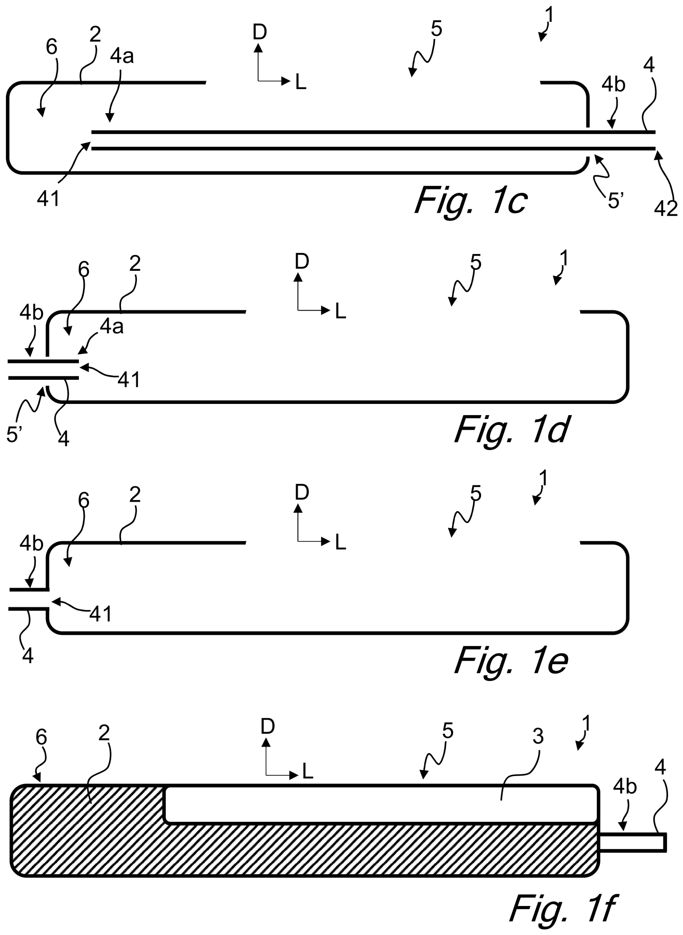

Alternatively, as shown in fig. 1c, it is envisaged that the liquid impermeable layer 2 is provided with an additional opening 5', separate from the urine inlet opening 5, to allow passage of the first portion of the conduit 4 into the interior volume.

The conduit 4 is configured to be connected to a vacuum source so as to provide a suction force through the conduit 4. Hereby, urine which enters into the interior volume via the urine inlet opening 5 is also pulled by the suction force towards the urine collection region 6 and thereafter into the conduit 4. By withdrawing urine via the conduit 4, it is possible to use the urine collection device 1 for a long period of time as urine may be continuously removed from the interior volume.

Further alternative embodiments are shown in figs. 1d and 1e. The conduit 4 may e.g. enter into the interior volume via the bottom portion 1a of the urine collection device 1 so as to directly communicate with the urine collection region 6 as seen in fig. 1d. This may enable the first portion 41 of the conduit 4 to be made shorter, since it does not have to extend past the urine inlet opening 5 in the top portion 1b.

It is also envisaged that in some embodiments the conduit 4 is terminated flush with the impermeable layer 2 in the bottom portion 1a wherein the conduit does not feature a first portion extending inside the interior volume, as shown in fig. 1e. A benefit with this arrangement is that the first opening 41 of the conduit 4 may form the bottom most part of the urine collection region 6, allowing the urine collection region 6 to be fully drained from urine.

Turning back to fig. 1a, the urine collection device 1 further comprises an absorbent material 3 arranged inside the interior volume. The absorbent material 3 extends from the urine inlet opening 5 towards the urine collection region 6 and defines a low flow urine transportation path. The absorbent material 3 may extend all the way to the urine collection region 6 or it may end upstream of the urine collection region 6. Additionally, a high flow urine transportation path 7 is also formed inside the interior volume. The high flow urine transportation path 7 is free from absorbent material and extends from the urine inlet opening 5 to the urine collection region 6. The two transportation paths may be realized in various ways, a number of which are described below, however by providing two transportation paths, the urine collection device 1 becomes capable of transporting urine even if the urine flow varies. Generally, the high flow urine transportation path 7 has the capacity to transport large urine flows and the low flow urine transportation path provides absorption capability to prevent leakages for slower flows. Preferably, the high flow urine transportation path 7 goes from the urine collection region 6 to the urine inlet opening 5. However, the low flow urine transportation path may communicate directly with the urine inlet opening 5 and be arranged to drain into the high flow urine transportation path 7 which extends the remaining distance to the urine collection region 6.

Fig. 1f shows a side view of the urine collection device 1 of fig. 1c. The absorbent material 3 covers a portion of the urine inlet opening 5. Hereby, when the urine collection device 1 is placed against the urethral opening of the user, any urine expelled from the user will reach the absorbent material 3, which absorbs the urine. Since the absorbent material 3 extends towards the urine collection region 6 and/or drains into the high flow urine transportation path 7, the urine will also be transported by the absorbent material 3 towards the conduit 4 by liquid transport mechanisms inherent to the absorbent material 3 (capillary forces, wicking), by gravity, and/or by the suction force originating from the vacuum source connected to the conduit 4.

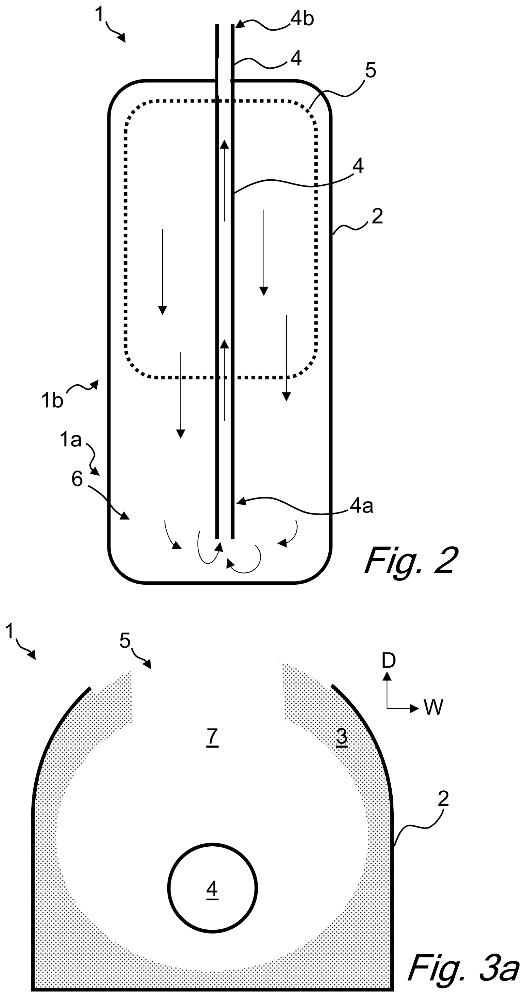

Fig. 2 illustrates schematically a length-width cross-section of the urine collection device 1 with the urine inlet opening 5 overlayed. The general transportation path of urine through the urine collection device 1 is also illustrated by multiple arrows. The urine enters through the urine inlet opening 5, flows towards the urine collection region 6, and is withdrawn from the urine collection region 6 using the conduit 4.

Turning back to fig. 1a, the urine collection device 1 comprises a high flow urine transportation path 7 interfacing with the urine inlet opening 5. The absorption material 3 also interfaces with the urine inlet opening 5 and defines a low flow urine transportation path. The two urine transportation paths extend from the urine inlet opening and towards the collection region 6 so as to enable urine entering through the urine inlet opening 5 to be transported via one or both paths to the urine collection region 6 and be withdrawn therefrom via the conduit 4. The low flow urine transportation path filled with the absorbent material is beneficial for preventing leakage but is more prone to saturation if the urine flow is high. The high flow urine transportation path 7, on the other hand, is not filled with an absorbent material and is capable of efficiently transporting larger flow rates of urine to the collection region 6. With both transportation paths interfacing with the urine inlet opening 5 a urine collection device 1 capable of handling large flows while also preventing leakages is realized.

Fig. 3a illustrates schematically a cross-section of the urine collection device 1 shown in fig. 1a along a width-depth plane P1 in the top portion 1b. The absorbent material 3 forming the low flow urine transportation path extends to the urine inlet opening 5 formed in the liquid impermeable layer 2. A space without absorbent material (i.e. an air-filled space) forms the high flow urine transportation path 7, allowing urine entering through the urine inlet opening 5 to flow towards the urine collection region without being obstructed by the absorbent material 3. It is envisaged that depending on the desired urine collection properties, the amount of space occupied by the high flow urine transportation path 7 with respect to the low flow urine transportation path (i.e. the absorbent material 3) may vary. In fig. 3a the interior volume is lined with the absorbent material 3 to leave a comparatively large high flow urine transportation path 7. This design is beneficial since it is capable of handling large urine flows, and a comparatively small amount of absorbent material 3 is sufficient for preventing leakages, as long as the absorbent material interfaces with the urine inlet opening 5.

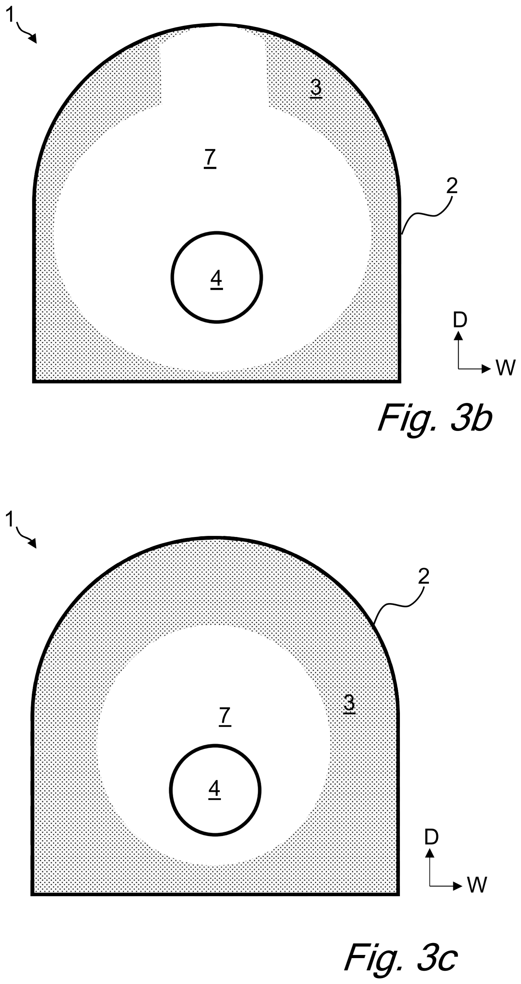

In fig. 3b, another cross-section in a width-depth plane P2 of the urine collection device 1 of fig. 1 is shown. Plane P2 is in the bottom portion 1a and closer to the bottom end of the urine collection device 1 compared to plane P1. Plane P2 does not intersect with the urine inlet opening 5. As seen, the high flow urine transportation path 7 continues along the conduit 4 towards the urine collection region 6. In some implementations, the high flow urine transportation path 7 maintains its cross-sectional shape as it extends along the elongation axis. Alternatively, the high flow urine transportation path 7 may change shape.

Fig. 3c shows an alternative high flow urine transportation path 7 in plane P2. As seen in this embodiment, the high flow urine transportation path 7 has a rounded cross-section which follows and surrounds the conduit 4. The high flow urine transportation path 7 is in turn surrounded by the absorbent material 3 allowing the absorbent material 3 to be drained.

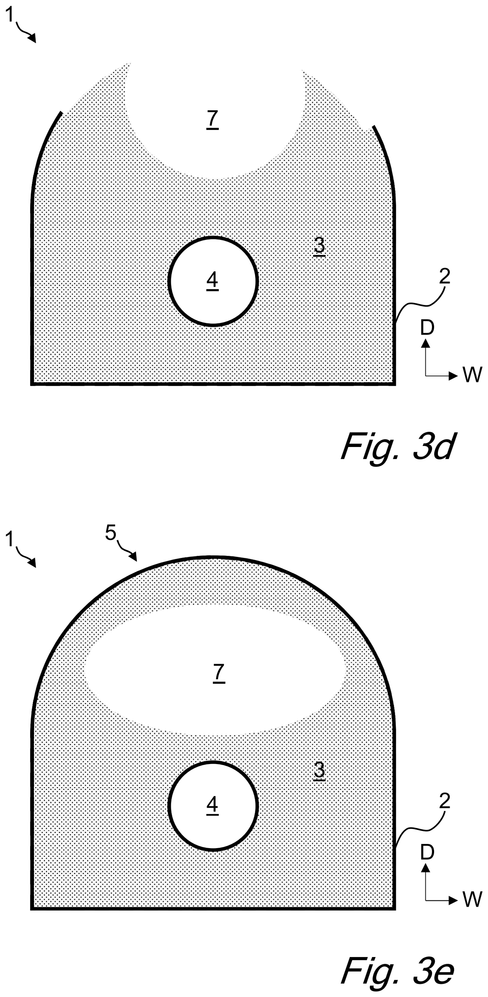

The relative placement and shape of the low flow urine transportation path and high flow urine transportation path 7 shown in figs. 3a-c are merely exemplary, and other placements and shapes are envisaged. Furthermore, while the high flow urine transportation path 7 reaches and follows the outside of the conduit 4 in figs. 3a-c this merely exemplary and it is envisaged that the high flow urine transportation path 7 may be displaced from the conduit both at the urine inlet opening 3 (e.g. at plane P1) and when approaching the urine collection region 6 (e.g. at plane P2) as shown in figs. 3d and 3e respectively.

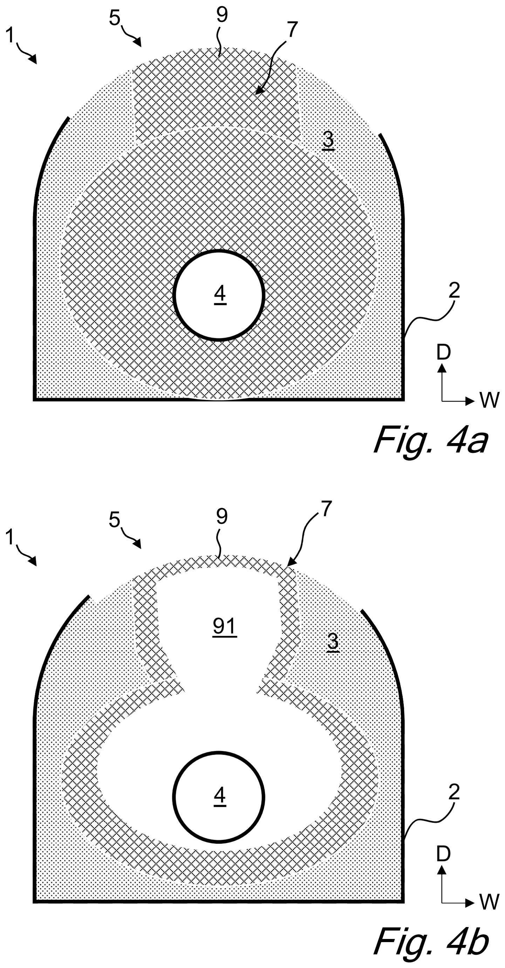

In some implementations, the high flow urine transportation path 7 is at least partially filled with a porous material 9, as shown in fig. 4a showing a cross section (e.g. at plane P1 overlapping with the urine inlet opening 5). The porous material 9 may comprise pores extending through the material, allowing the urine to flow through the porous material. For example, the porous material 9 is a large pore synthetic sponge material or a 3D mesh material. The porous material 9 may be rigid or soft and contributes to keeping the high flow urine transportation path 7 from collapsing. As further examples, the porous material 9 is a skeletonized synthetic sponge made of e.g. a polymer material such as plastic. As another example, the porous material comprises a plurality of loose bodies (e.g. beads or pellets of regular or irregular size) which help define the high flow urine transportation path by keeping the absorbent material away while at the same time allowing urine to pass rapidly through the space between the bodies. As with an air-filled high flow urine transportation path, the porous material 9 may allow drainage of the absorbent material 3 at any point where these two-materials interface. This allows the absorbent material 3 to not necessarily extend all the way to the urine collection region as the urine may be drained into the high flow urine transportation path 7.

In some implementations, the porous material 9 is shaped into a hollow tube shape having an exterior wall made of the porous material 9 (e.g. sponge material or 3D mesh material) and central channel 91, one example of which is shown in fig. 4b. The porous material 9 may in this implementation hold the high flow urine transportation path 7 open and the central channel 91 further enhances urine flow capability since urine may enter the central channel 91 via the porous walls and subsequently rapidly approach the urine collection region.

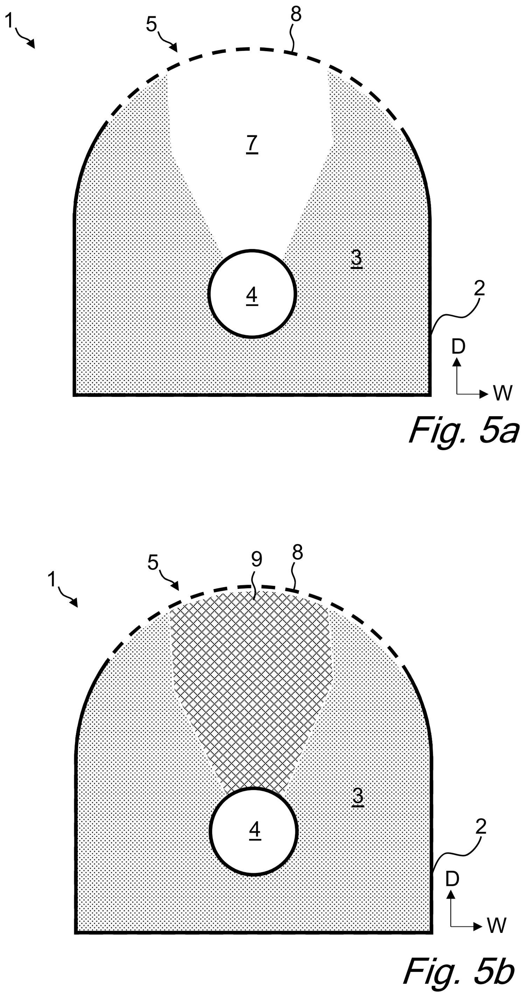

In some implementations, at least a portion, or the entirety, of the urine inlet opening 5 is covered with a permeable material 8, as will now be described in connection to figs 5a-c.

In fig. 5a, an empty space (air filled) high flow urine transportation path 7 and a low flow urine transportation path (realized with the absorbent material) are covered at the urine inlet opening 5 with a layer of permeable material 8. The permeable material 8 may be a net or similar configured to allow urine to pass through easily. For example, the openings of the permeable material may have an opening dimension large enough to allow urine to pass through, but still small enough not to be uncomfortable when held against the skin of the user. For example, the largest dimension of the openings is 1 mm or less, 0.8 mm or less, or 0.5 mm or less. The permeable material 8 may contribute to keeping a desired shape of the urine collection device 1 and/or may help keeping the high flow urine transportation path 7 open.

The cover made of permeable material 8 for the urine inlet opening 5 may be used with any type of high flow urine transportation path 7, such as a high flow urine transportation path 7 defined by empty space or a high flow urine transportation path 7 defined by a porous material 9 as shown in fig. 5b. In the former case, another benefit with providing a permeable cover 8 is that this cover 8 may prevent body parts of the user from entering into the high flow urine transportation path 7 where there otherwise could be a risk of user injury by getting a body part stuck or pinched inside the urine collection device 1.

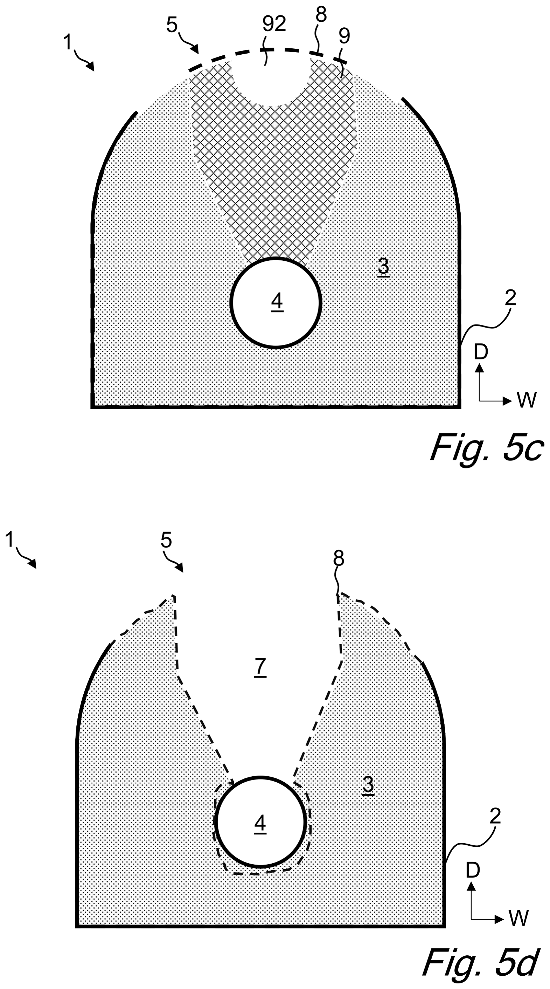

In some implementations, the cover made of permeable material 8 is arranged to cover the high flow urine transportation path 7 while leaving the typically soft and comfortable absorbent material 3 exposed, as shown in fig. 5c. In this way, the urine collection device 1 may be safely held against a user while at the same time urine can pass into the high flow urine transportation path 7 via the cover of permeable material 8. Fig. 5c also shows that the porous material 9 may be provided with a groove 92, further increasing the fluid flow rate. With the aid of a groove 92 facing the urine inlet opening 5, the urine that passes the permeable material will interface with the porous material 9 over a larger area (in the groove), which will facilitate leading urine into the channels through the porous material 9.

In some implementations, the permeable material 8 is arranged to wrap the absorbent material 3 and helps maintain a desired shape of the absorbent material 3 and/or helps define the high flow urine transportation path 7, as illustrated in fig. 5d. In some implementations, the absorbent material 3 is at least partially separated from the high flow urine transportation channel 7 with the permeable material 8.

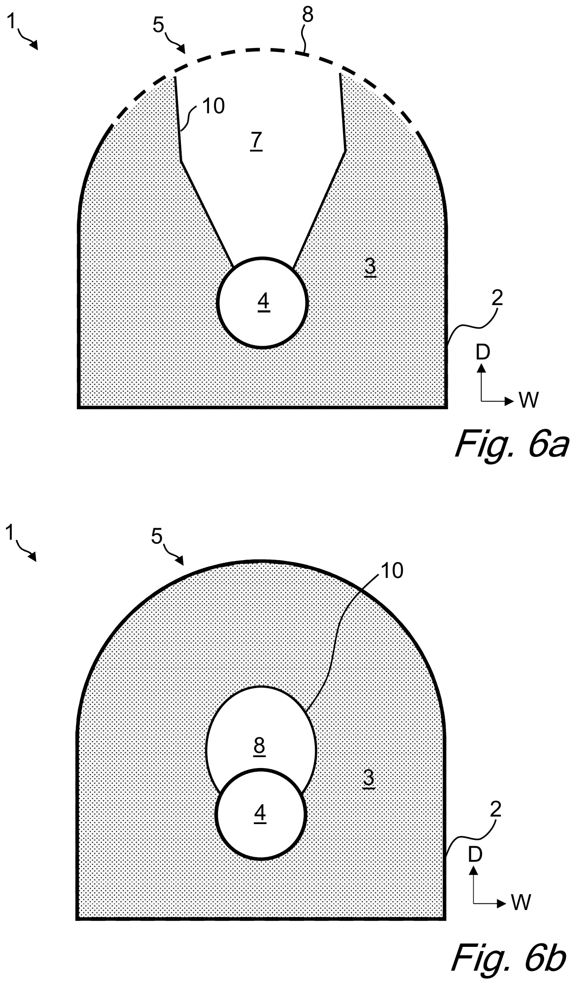

In some implementations, a urine guiding body 10 is provided inside the interior volume to at least partially define the high flow urine transportation path 7 as shown in figs. 6a and 6b. The urine guiding body 10 may be made of a liquid permeable or liquid impermeable material. For example, the urine guiding body 10 is made of a liquid impermeable polymer material such as a plastic material. The urine guiding body 10 may be rigid or elastically deformable. For example, the urine guiding body 10 is made of an elastomer material or a material with shape memory. In embodiments where the urine guiding body 10 is made of a liquid impermeable material, the urine guiding body 10 may be provided with slits or openings to allow urine to enter the high flow transportation path at various points along the extent of the urine guiding body, to e.g. allow the absorbent material 3 to drain into the high flow urine transportation path 7.

In fig. 6a, a cross-section in a width-depth plane, overlapping with the urine inlet opening 5 of a urine collection device with a urine guiding body, is shown. The urine guiding body 10 forms a channel or trough that at least partially defines the high flow urine transportation path 7. The urine guiding body 10 may extend all the way to the urine inlet opening 5. The urine inlet opening 5 may be configured to allow urine entering through the urine inlet opening 5 to enter either the high flow urine transportation path 7 or the absorbent material 3 defining the low flow urine transportation path.

In some implementations, the urine guiding body 10 comprises a receptacle configured to collect urine at the urine inlet opening 5 and guide the urine to the high flow urine transportation 7 path at least partially defined by the urine guiding body 10. The receptacle may act as a funnel and may be formed as a tapered inlet or other type of fluid collector.

Moving away from the cross-section of fig. 6a, along the elongation/length axis and towards the bottom end of the urine collection device 1, the schematic cross-section of fig. 6b is encountered in some implementations with a urine guiding body 10. As seen, the urine guiding body 10 has transitioned into a semi-tubular shape that contacts the outside of the conduit 4 to form the high flow urine transportation path 7. Accordingly, as described above, the urine guiding body 10 may have a receptacle interfacing with the urine inlet opening 5 which is comparatively wider than a narrower path defined partially by the urine guiding body 10, as the urine guiding body 10 approaches the urine collection region. Hereby, the receptacle of the urine guiding body 10 acts as a funnel or fluid collector for guiding the urine into the high flow urine transportation path 7.

In figs. 6a and 6b, the urine guiding body 10 is attached to, or directly abutting, the conduit 4. However, it is understood that this is merely exemplary, and embodiments are envisaged where the urine guiding body 10 is arranged separately from the conduit 4. Furthermore, while the urine guiding body 10 is depicted as a solid element defining a solid, e.g. liquid impermeable, path along at least a portion of its extent, it is realized that the urine guiding body 10 may be provided with one or more openings or even be realized from a mesh material. The urine guiding body 10 may at least act as a scaffolding serving to hold open the high flow urine transportation path 7 along at least a portion of the elongation length of the urine collection device 1.

The urine guiding body 10 may, in addition to at least partially defining the high flow urine transportation path 7, contribute to supporting a desired shape of the urine collection device 1. In some implementations, the liquid impermeable layer 2 and the absorbent material 3 are soft and deformable, whereby it is only the conduit 4 which provides some structure to the urine collection device 1. However, the urine guiding body 10 may contribute to retaining the urine collection device 1 in a desired shape, such as a generally cylindrical, rectangular cuboid or composite solid shape. The urine guiding body 10 may also be configured to retain a generally curved shape which by extension forms a curvature for the urine collection device 1, making it more comfortable and/or making it better adapted for close contact with the region surrounding the urethra of the user, which also is generally curved. In some implementations (e.g. when no urine guiding body is provided), the shape of the urine collection device 1 is defined primarily by the porous material defining the high flow urine transportation path. Additionally or alternatively, it is understood that the impermeable layer wrapping the absorbent material and/or porous material and/or the conduit may contribute to defining the shape of the urine collection device.

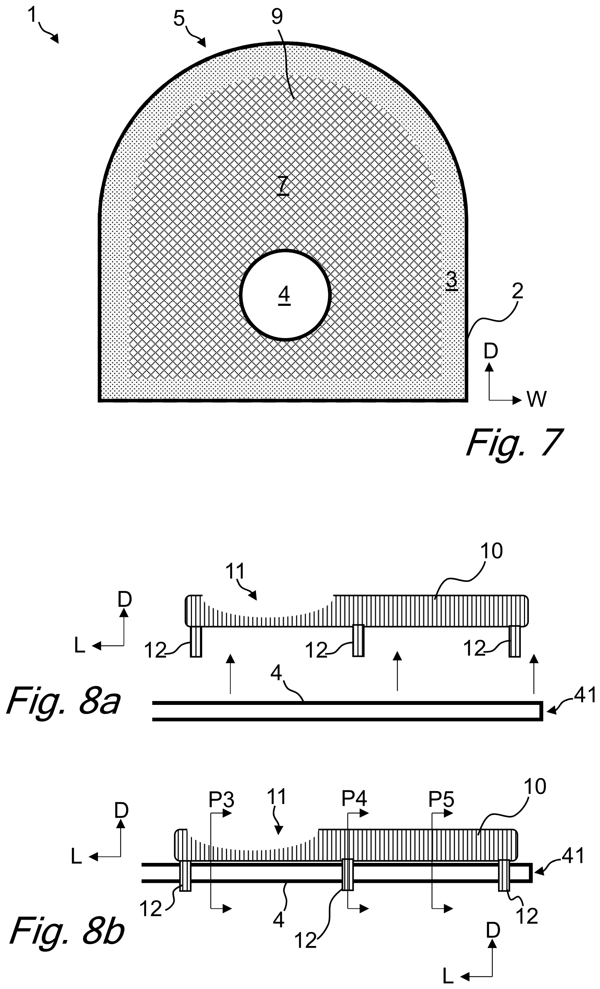

Fig. 7 shows a cross-section in the length-width plane P2 (see fig. 1a) of a urine collection device according to some implementations. In this implementation, the high flow urine transportation path 7 is realized using a porous material 9, and the absorbent material 3 is in the form of a lining which is wrapped around the high flow urine transportation path 7 and at least a portion of the conduit 4. In this implementation, the porous material 9 at least partially defines the high flow urine transportation path 7 and interfaces with the absorbent material 3 over a large surface area, whereby the high flow urine transportation path 7 may contribute to draining the absorbent material 3 from urine. It is understood that while the high flow urine transportation path 7 of fig. 7 is at least partially formed with a porous material 9, other elements could be used to form the high flow urine transportation path 7, such as a urine guiding body which supports the absorbent material 3 lining. To facilitate drainage of the absorbent material 3, the urine guiding body may be provided with a plurality of openings allowing urine to escape from absorbent material 3 into the high flow urine transportation path 7 at least partially defined by the urine guiding body.

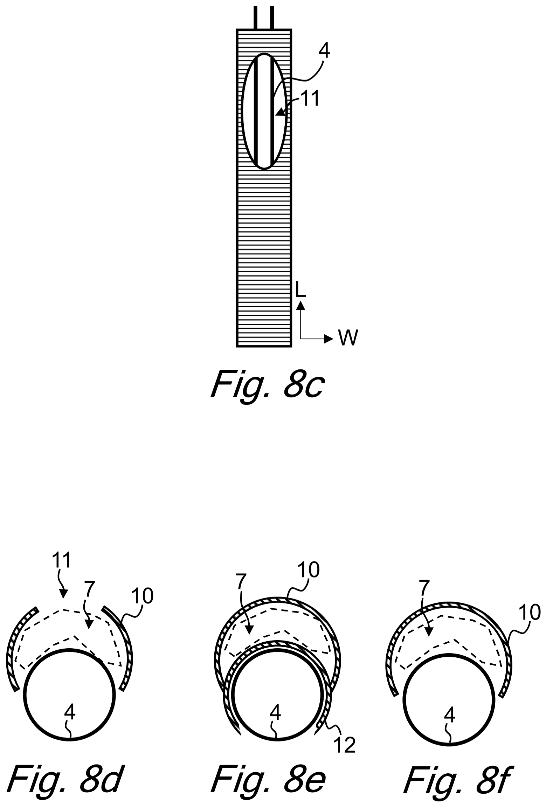

Figs. 8a-c shows an exemplary urine guiding body 10 according to some implementations. The urine guiding body 10 comprises one or more (in the present case three) attachment elements 12 for attaching the guiding body 10 to the conduit 4. The attachment elements 12 may be configured to snap around the conduit 4 or formed as rings allowing the conduit 4 to be inserted through the rings so as to be attached to the urine guiding body 10.

The urine guiding body 10 has an opening 11 arranged so as to communicate with the urine inlet opening of the liquid impermeable layer. Hereby, when the urine guiding body 10 is attached to the conduit 4, urine entering through the urine inlet opening may enter through the opening 11 and reach the high flow urine transportation path at least partially defined by the urine guiding body 10.

Figs. 8d-f show cross-sectional views at various depth-width planes P3, P4, P5 along the elongation axis of the conduit 4 and the urine guiding body 10. In fig. 8d the cross-section P3 (see fig. 8b) is shown overlapping with the opening 11 in the urine guiding body 10. As seen, urine may enter through the opening 11 to reach the high flow urine transportation path 7 at least partially defined by the urine guiding body 10 (and in this case also the outer surface of the conduit 4). With the aid of e.g. gravity and/or the suction pressure of the conduit 4, urine is transported along the high flow urine transportation path 7 towards the first opening 41 of the conduit 4. In fig. 8e, a cross-section P4 of fig. 8b, located downstream (i.e. towards the urine collection region) of cross-section P3, is shown. Here, an attachment element 12 configured to snap onto the conduit 4 is shown, and the attachment element 12 is attached to the remaining part of the urine guiding body 10 wherein the urine guiding body still at least partially defines a high flow urine transportation path 7 for leading urine to the collection region. In fig. 8f, a cross-section P5 of fig. 8b, located downstream (i.e. towards the urine collection region) of both cross-section P3 and P4, is shown. As seen, the urine guiding body 10 still at least partially defines the high flow urine transportation path 7 (together with the outer surface of the conduit).

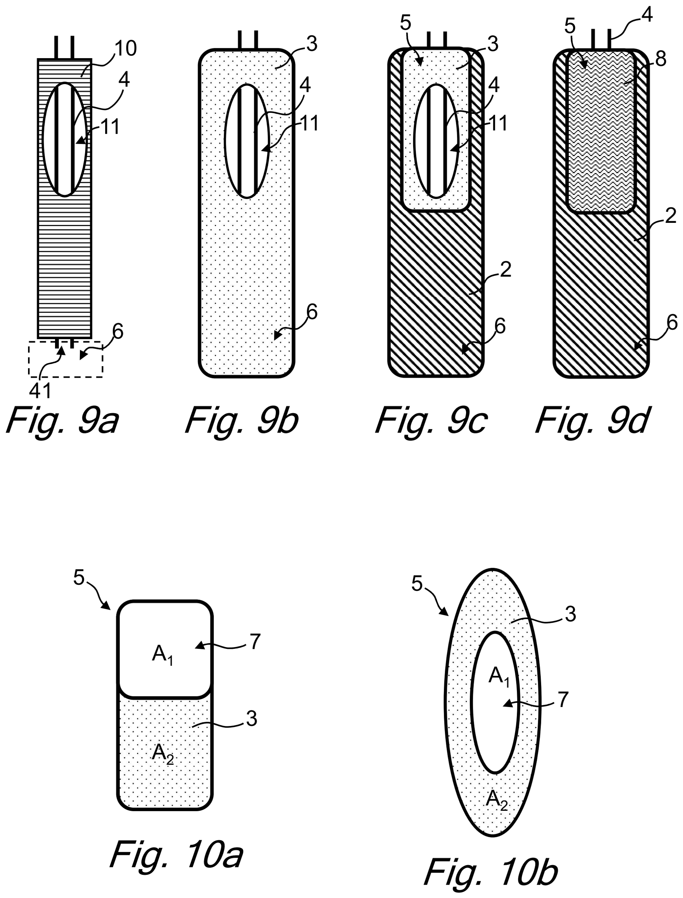

In fig. 9a, the urine guiding body 10 is illustrated mounted onto the conduit 4. Urine entering the opening 11 in the urine guiding body 10 may flow to the urine collection region 6, schematically illustrated below the urine guiding body 10, and in communication with the first opening 41 of the conduit 4. In some implementations, to ensure that the first opening 41 of the conduit 4 is not obstructed and that urine may be drained effectively, the urine collection region 6 is air-filled or filled with a porous material (i.e. the urine collection region 6 may form an extension or part of the high flow urine transportation path). To maintain an air-filled urine collection region a scaffolding and/or an extension of the urine guiding body 10 may be provided to keep the impermeable layer and/or absorbent material away from the first opening 41.

In fig. 9b, the absorbent material 3 has been added on top of the urine guiding body. The absorption material 3 may be shaped like a sleeve which is threaded over the conduit 4 and the urine guiding body 10, wherein the sleeve has a radial opening allowing the opening 11 of the urine guiding body 10 to be exposed. Alternatively, the absorbent material is 3 is shaped like a sheet which is wrapped around the conduit 4 and the urine guiding body while leaving the opening 11 of the urine guiding body 10 exposed.

In fig. 9c, the liquid impermeable layer 2, having the urine inlet opening 3, has been added so as to surround the parts of fig. 9b, namely the conduit 4, the urine guiding body 10 and the absorbent material 3. The urine inlet opening 5 is sized and adapted to enable direct fluid communication between the outside of the liquid impermeable layer 2 and both the absorbent material 3 (forming the low flow urine transportation path) and the opening 11 (acting as the inlet for the high flow urine transportation path). Although not seen in fig. 9b and 9c, it is understood that the impermeable layer 2 and absorbent material 3 forms a region acting as the urine collection region 6 so that components do not obstruct the flow into the first opening 41 of the conduit 4.

Optionally, as described in connection with figs. 5a-d above, a permeable layer 8 may be added to cover the urine inlet opening 3 as shown in fig. 9d. The permeable layer 8 is configured to not impede urine flow to a noticeable extent, and urine leaving the user may still reach each of the two fluid transportation channels via the permeable layer 8. In fig. 9d, the permeable layer 8 covers the urine inlet opening 5 completely, but it is understood that different arrangements of the permeable layer 8 could also be used. For example, the permeable layer 8 is arranged so as to only cover the absorbent material 3 (see for example fig. 5d) while leaving the inlet to the high flow urine transportation path exposed. A benefit with this arrangement is that the permeable layer 8 may be used to retain the soft and/or deformable absorbent material 3 and keep it out of the way of the inlet to the high flow urine transportation path. As another example, the absorbent material 3 is exposed and the permeable layer 8 is arranged only over the inlet to the high flow urine transportation path 7. For instance, the permeable layer 8 is attached to the porous material or urine guiding body at least partially defining the high flow urine transportation path 7. A benefit with the permeable layer 8 covering the inlet to the high flow urine transportation path 7 is that that no body part of the user can enter the high flow urine transportation path. In other words, the user will only contact the absorption material 3, the permeable layer 8 or the impermeable layer 2, whereas the user is prevented from coming into contact with e.g. the porous material or the urine guiding body.

In the urine collection device 1, shown in e.g. figs. 1 and 9c, the entry area into the high flow urine transportation path is surrounded by absorbent material 3. However, while there are some benefits associated with this disposition of the entry the high flow urine transportation path with respect to the absorbent material, other dispositions are envisaged. In fig. 10a one such alternative disposition is shown, where the entry the high flow urine transportation path 7 is offset from, but not surrounded by, the absorbent material 3. In some implementations, the entry into the high flow urine transportation path 7 is located above the absorbent material 3 when the urine collection device is in its use position. For example, the entry into the high flow urine transportation path 7 may be configured to face the urethral opening when the urine collection device is held against the user, whereas at least a portion of the absorbent material is located below the entry into the high flow urine transportation path 7 so as to absorb urine which travels along the skin of the user.

In fig. 10b, a layout similar to that of figs. 1 and 9c is shown, with the entry into the high flow urine transportation path 7 surrounded by the absorbent material 3. A benefit with this layout is that urine which e.g. travels along the skin of the user, or which misses the entry into the high flow urine transportation path 7 in any direction, is absorbed by the absorbent material 3 and transported to the urine collection region to prevent leakages. Still, the entry into the high flow urine transportation path 7 may be configured so as to face the urethral opening when held against the user such that e.g. rapid gushes of urine expelled by the user enters directly into the high flow urine transportation path 7, whereas slower, more continuous outflows of urine are absorbed by the absorbent material 3.

In figs. 10a and 10b, the area of the entry into the high flow urine transportation path 7 and area of the absorbent material 3 is labeled area A₁ and A₂. In some implementations, A₁ is smaller than A₂. For example, the area A₂ covered by the absorbent material 3 at the urine inlet opening 5 is at least 30%, at least 40% or at least 50% larger than the area A₁ of the entry into the high flow urine transportation path 7. Even though the area of the entry into the high flow urine transportation path 7 may be smaller, it features high urine transportation capacity since it is generally less obstructive for the urine than the absorbent material 3. On the other hand, if urine does not enter directly into the entry of the high flow urine transportation path 7, it may leak out from the urine collection device. To this end, the absorbent material 3 is used to absorb urine and prevent leakages. By having a larger area A₂ of the absorbent material 3, the leakage prevention effect will be enhanced at the same time as the risk of saturating the absorbent material 3 decreases.

Nevertheless, it is also envisaged that the area A₁ may be greater than the area A₂. For example, the area A₁ corresponding to the entry into the high flow urine transportation path 7 at the urine inlet opening 5 is at least 30%, at least 40% or at least 50% larger than the area A₂ covered by the absorbent material 3.

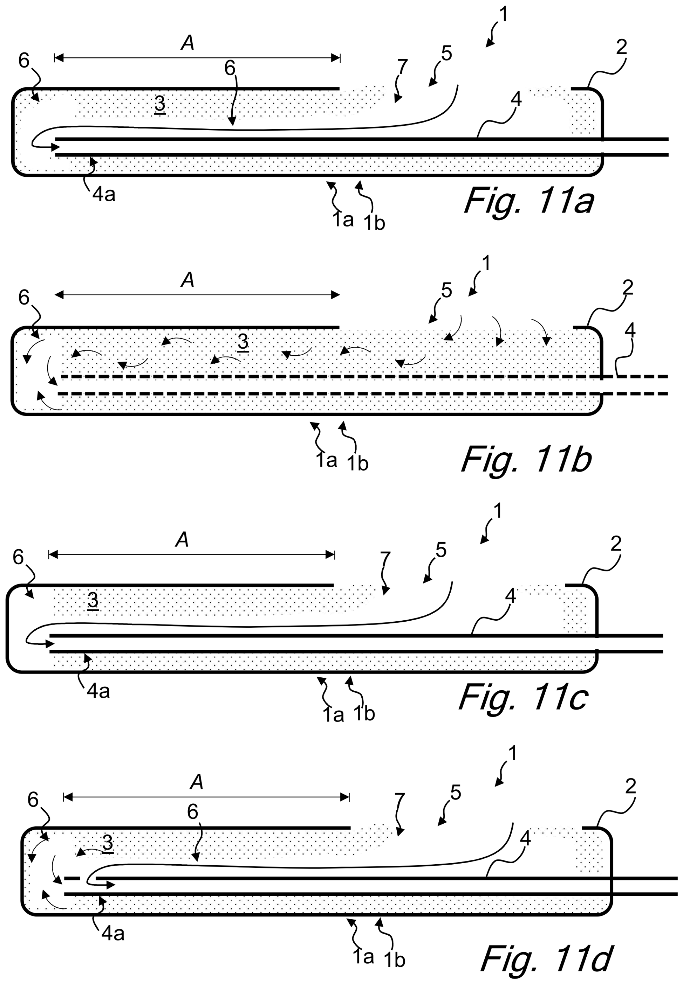

Fig. 11a shows a cross-section in a length-depth plane of a urine collection device 1 according to some implementations. Urine entering through the urine inlet opening 5 may travel towards the urine collection region 6 in the high flow urine transportation path 7 which is free from absorbent material 3. For example, the high flow urine transportation path 7 is held open using a porous material, using a urine guiding body and/or by merely an absorbent material 3 shaped/formed so as to leave an air-filled high flow urine transportation path 7.

According to one definition, the high flow urine transportation 7 extends in the upper portion 1b of the urine collection device 1. The upper portion 1b of the urine collection device 1 transitions to the lower portion at the lower end of the urine inlet opening 5. At this point along the elongation axis, the high flow urine transportation path 7 transitions into the urine collection region 6. The high flow urine transportation path 7 and urine collection region 6 are both free from absorbent material 3 and defined by one or more of an air-filled space, a urine guiding body, a porous material and the impermeable layer 2 whereby it is possible for urine to reach the conduit via a path with low flow resistance. The absorbent material 3 may drain into one or both of the urine collection region 6 and the high flow urine transportation path 7.

In some implementations, the urine collection region 6 extends along at least a portion of the elongation axis of the urine collection device 1 as shown in fig. 11a. The length A over which the urine collection region 7 extends may be at least 3 cm, at least 5 cm or at least 6 cm. The high flow urine transportation path 7 may transition into the urine collection region 6. That is, the high flow urine transportation path 7 may serve as an extension of the urine collection region 6 providing more space for urine to temporarily accumulate as it flows towards the first opening of the conduit. A sufficiently large (e.g. volume, length and/or diameter) high flow urine transportation path 7 and/or urine collection region 6 means that the risk of urine filling up all the way to the urine inlet opening 5 is reduced and that the urine collection device 1 is less sensitive to how it is tilted.

As described above, in addition to urine passing through the high flow urine transportation path 7, the urine may be absorbed by the absorbent material 3 and transported through the absorbent material 3 by wicking, gravity and/or suction. Fig. 11b shows another cross-section in a length-depth plane offset from the length-depth plane used for the cross-section in fig. 11a. For example, this cross-section may be to one side of the conduit 4 (imaginary projection shown). In this region, there may be no high flow urine transportation path and urine is mainly transported via the absorbent material 3 towards the urine collection region 6. The absorbent material 3 may be drained into the high flow urine transportation path and/or urine collection region 7 along the way towards the urine collection region 6. Preferably, the urine collection region 6 is air-filled as shown in figs. 11a-c, filled with a porous material and or defined by a urine guiding body. A benefit with at least partially filling the urine collection region 6 with a porous material and/or urine guiding body is that this may prevent the impermeable layer 2 or absorbent material 3 from collapsing and blocking the opening(s) of conduit 4.

Additionally or alternatively, the conduit 4 comprises at least two openings, e.g. as shown in fig. 11d or by the addition of a manifold, wherein each opening communicates mainly with a respective one of the high flow urine transportation path 7 and the absorbent material 3, wherein each opening communicates with a same transportation path, such as the high flow urine transportation path 7, and/or wherein the multiple openings all communicate with the urine collection region 6.

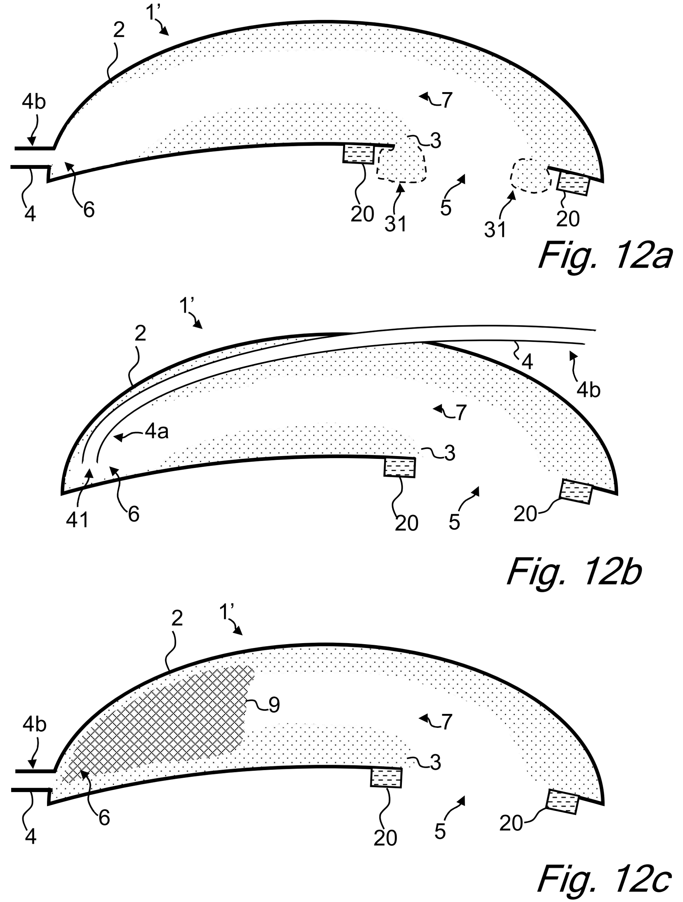

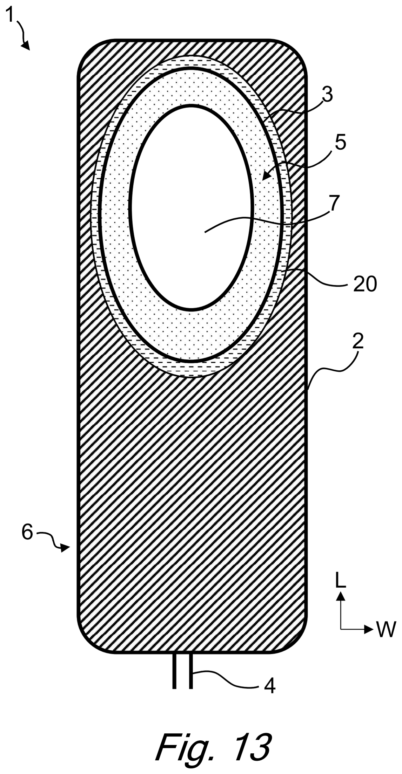

The urine collection device described above may be especially well suited for female users or male users with a retracted or buried penis. However, the same principles may be used to form a urine collection device suitable for male users or any user with a penis, as will now be described in connection with figs. 12a-c and 13.

Fig. 12a illustrates a urine collection device 1' specifically adapted for male users (or in general any user with a penis). The urine collection device 1' is similar to the urine collection device 1 described in terms of it featuring a high flow urine transportation path and a low flow urine transportation path defined by the absorbent material 3. The high flow urine transportation path 7 of the urine collection device 1' does, however, start at the urine inlet opening 5 with an air-filled upstream portion towards the urine transportation path. The air filled channel is sized and adapted to receive at least a portion of a penis allowing the penis to extend into the high flow urine transportation path 7.

The urine collection device 1' may further be configured such that downstream the upstream portion, in a downstream portion of the high flow urine transportation path 7, the high flow urine transportation path 7 (and optionally low flow urine transportation path) continue towards the urine collection region 6 which communicates with the conduit 4.

In some implementations, the low flow urine transportation path (i.e. the absorbent material 3) is configured to extend along the penis. Additionally or alternatively, the absorbent material 3 is arranged to circumferentially surround the urine inlet opening 5 (and surround the shaft of a penis when the urine collection device is in use) to absorb urine moving along the shaft of the penis when in use. As a further option, the inlet opening 5 is configured to receive both the shaft of a penis and the scrotum, wherein the absorbent material 3 surrounds the base of both the penis and the scrotum.

With an absorbent material surrounding the base of the penis or the base of the penis and scrotum, leakages may be effectively prevented, leakages which otherwise may occur if the urine inlet is not completely lined with the absorbent material. The absorbent material 3 may line the interior surface of the impermeable layer 2 and extend along the upstream portion and along at least a part of the downstream portion of the high flow urine transportation path 7.

In some implementations, the absorbent material protrudes out from the urine inlet opening 5 and extends along the exterior surface of the impermeable layer 2 in proximity to the urine inlet opening 5. In this way, the absorbent material 3 forms a collar 31 surrounding the urine inlet opening 5 as shown in fig. 12a. A collar 31 may both improve comfort and improve absorption performance by making sure that the absorbent material 3 contacts the skin of the user.

In fig. 12a, the conduit 4 enters the interior volume directly at the urine collection region 6. However, this is merely exemplary and shown in fig. 12b the conduit 4 may enter the interior volume at some other location (in general at any location) and extend to reach and communicate with the urine collection region 6.

In the embodiment shown in fig. 12b the conduit enters into the interior volume and extends along the empty space configured to receive a penis to reach the urine collection region 6 downstream of the upstream portion of the high flow urine transportation path. In general, it is not a problem if the conduit 4 contacts the penis when the penis is inserted, however, to make the urine collection device 1' more comfortable to use the conduit may be embedded in the absorbent material 3 whereby the conduit 4 is not directly contacted by the user.

It is understood that any of the above mentioned variants of the high flow urine transportation path, such as a high flow urine transportation path at least partially filled with an absorbent material and/or at least partially defined with a urine guiding body may be used in the urine collection device 1' adapted for males. The main difference being that the urine collection device 1' comprises an upstream portion of the high flow urine transportation path which is air-filled, and a downstream part of the high-flow urine transportation path is provided with a urine guiding body and/or porous material at least partially defining the downstream part of the high flow urine transportation path 7. It is also envisaged that the upstream portion comprises a urine guiding body sized and adapted to receive the shaft of a penis and/or a porous material with a channel, the channel being sized and adapted to receive the shaft of a penis.

Fig. 12c shows one embodiment wherein a porous material 9 is provided in the downstream part of the high flow urine transportation path 7. A benefit with this design is that the porous material 9 keeps the impermeable layer from collapsing by keeping the device open so as to avoid undesirable restriction of the flow to the conduit 4.

In some implementations, the urine collection device 1' is provided with an attachment element 20 surrounding the urine inlet opening and allowing the urine collection device 1' to be attached to the user. The attachment element 20 may e.g. be an adhesive or similar allowing the urine collection device to be temporarily attached to the area surrounding the base of the penis. This helps to keep the urine collection device 1' in place for long time use.

Of course, an absorbent material 3 extending outside the urine inlet opening 5 to form a cuff 31 as shown in fig. 12a may be used in the embodiments of fig. 12b-c.

Fig. 13 shows a front view of the urine collection device 1' for male users. The air-filled first part of the high flow urine transportation path 7 is visible. At the urine inlet opening, the absorbent material 3 surrounds the high flow urine transportation 7 to absorb any urine flowing backwards when the urine collection device 1' is used. This may help prevent urine leakage. Additionally, the urine collection device 1' is provided with an attachment element 20 for attaching the urine collection device 1' to the skin of the user. The attachment element 20 may be an area of adhesive which surrounds the urine inlet opening 3.

Due to the absorbent material 3 surrounding the urine inlet opening 5 it is also envisaged that the urine collection device 1' may be secured over the shaft of a penis or over the base of the penis and scrotum by other means. For example, the absorbent material 3 (and especially when a collar of absorbent material is provided) will absorb urine even if the urine collection device 1' is not adhered directly to the skin of the user. Other types of attachment, which per se are not liquid tight such as a rubber band or string, may be used to gently secure the urine collection device 1' to the user. As long as the absorbent material 3 contacts the user preferably circumferentially around the base of the penis, or penis and scrotum, it will absorb urine and form a comfortable and effectively liquid tight seal.

The impermeable layer 2 traps any urine expelled by the user when the urine collection device 1' is used and the urine is transported away via the conduit 4.

A person skilled in the art realizes that the present invention by no means is limited to the preferred embodiments described above. On the contrary, many modifications and variations are possible within the scope of the appended claims. For example, various ways for realizing the high-flow urine transportation path have been presented, such as using the absorbent material to form an empty (air filled) path, using a porous material or using a urine guiding body, and it is understood that these may be combined in various ways to form the high flow urine transportation path. For example, a porous material may be arranged inside the urine guiding body or a urine guiding body may be used to define a first portion of the high flow urine transportation path whereas a second portion is formed by a porous material or an absorbent material arranged to form an empty space. Any features described in connection to a urine collection device intended for female users or users with a retracted or buried penis may be provided in a corresponding manner in a urine collection device configured for male users or users with a penis.

In the claims, the word "comprising" does not exclude the presence of other elements or steps than those listed in the claim. The word "a" or "an" preceding an element does not exclude the presence of a plurality of such elements.

## Claims

1. A urine collection device comprising:
a liquid impermeable layer at least partially defining an interior volume, wherein the liquid impermeable layer comprises a urine inlet opening and a collection region for urine,
a conduit comprising a first opening, wherein the first opening is in communication with the collection region of the interior volume, and
an absorbent material arranged inside the interior volume and extending from the urine inlet opening towards the collection region so as to define a low flow urine transportation path,
wherein the urine collection device further comprises a high flow urine transportation path extending from the urine inlet opening to the collection region, wherein a urine flow rate per unit area is higher for the high flow urine transportation path compared to the low flow urine transportation path.

2. The urine collection device according to claim 1, wherein the high flow urine transportation path is at least partially filled with a porous material.

3. The urine collection device according to claim 2, wherein the porous material is non-absorbent, wherein the collection region is at least partially filled with the porous material.

4. The urine collection device according to any one of claims 1 to 3, wherein the liquid impermeable layer comprises an impermeable film.

5. The urine collection device according to claim 4, wherein the impermeable film is laminated with a nonwoven material on at least one of an exterior surface of the impermeable film and an interior surface of the impermeable film.

6. The urine collection device according to any one of claims 1 to 5, wherein the high flow urine transportation path is at least partially defined by an air-filled space.

7. The urine collection device according to any one of claims 1 to 6, further comprising:
a urine guiding body arranged inside the interior volume, the urine guiding body forming a channel at least partially defining the high flow urine transportation path.

8. The urine collection device according to claim 7, wherein a first portion of the conduit extends through the interior volume and wherein the urine guiding body is attached to the first portion of the conduit and extends at least partially along the first portion of the conduit.

9. The urine collection device according to claim 7 or 8, wherein the urine guiding body comprises a receptacle arranged to face the urine inlet opening, wherein the receptacle is configured to direct urine entering the receptacle to the channel.

10. The urine collection device according to any one of claims 1 to 9, wherein the urine inlet opening is at least partially covered with a permeable layer, wherein the urine inlet opening is surrounded by an attachment element comprising an adhesive configured for attaching the urine collection device to a user.

11. The urine collection device according to claim 10, wherein the permeable layer comprises a plurality of openings, wherein each opening has a maximum dimension of 1 mm or less, or 0.8 mm or less.

12. The urine collection device according to any one of claims 1 to 11, wherein an interface between the low flow urine transportation path is covered with a permeable layer, wherein the permeable layer comprises an opening and wherein the high flow urine transportation path communicates with the opening of the permeable layer.

13. The urine collection device according to any one of claims 1 to 12, wherein a permeable layer covers an interface between the high flow urine transportation path and the urine inlet opening and wherein a remaining area of the urine inlet opening is covered with the absorbent material.

14. The urine collection device according to any one of claims 1 to 13, wherein the absorbent material comprises an absorbent layer at least partially surrounding the high flow urine transportation path, wherein the absorbent material is a wicking material.

15. The urine collection device according to any one of claims 1 to 14, wherein the high flow urine transportation path is at least partially surrounded by the low flow urine transportation path at the urine inlet opening, and wherein the high flow urine transportation path comprises an air-filled upstream portion and a downstream portion connecting to the collection region, wherein the air-filled upstream portion is configured to receive a shaft of a penis and wherein the downstream portion is at least one of:
air-filled,
at least partially filled with a porous material, and
comprises a urine guiding body.
